# FASCICULE DE BREVET EUROPEEN

(11) **EP 3 331 064 B1**
(45) Date de publication et mention de la délivrance du brevet: **17.03.2021**
(21) Numéro de dépôt: 17203798.8
(22) Date de dépôt: 27.11.2017
(51) Int. Cl.: H01M 4/04, H01M 4/38, H01M 4/60, H01M 10/0525, H01M 10/054, C07D 233/26, H01M 4/62, H01G 11/00

(54) **UTILISATION DE L'ACIDE 4,5-IMIDAZOLEDICARBOXYLIQUE COMME MATÉRIAU ACTIF D'ÉLECTRODE**
ANWENDUNG VON 4,5-IMIDAZOLDICARBOXYLSÄURE ALS AKTIVES MATERIAL EINER ELEKTRODE
USE OF 4,5-IMIDAZOLEDICARBOXYLIC ACID AS AN ELECTRODE ACTIVE MATERIAL

(30) Priorité: 01.12.2016 FR 1661790
(43) Date de publication de la demande: 06.06.2018
(73) Titulaire: Commissariat à l'Energie Atomique et aux Energies Alternatives, 75015 Paris (FR)
(72) Inventeur: PERALTA, David, 38000 GRENOBLE (FR); GUTEL, Thibaut, 38113 VEUREY-VOROIZE (FR); LIONS, Mathieu, 3800 GRENOBLE (FR)
(74) Mandataire: Nony

(56) Documents cités:
- CN-A- 103 236 542
- FR-A1- 2 965 107
- COOPER G ET AL: "Reaction of dimethyl imidazole-4,5-dicarboxylate with styrene oxide", ROYAL CHEMICAL SOCIETY. JOURNAL. PERKIN TRANSACTIONS 1, ROYAL SOCIETY OF CHEMISTRY, GB , no. 9 1 janvier 1975 (1975-01-01), pages 798-803, XP008183055, ISSN: 1472-7781, DOI: 10.1039/P19750000798 Extrait de l'Internet: URL:http://pubs.rsc.org/en/content/article pdf/1975/p1/p19750000798
- KENNETH M. HARMON ET AL: "Hydrogen bonding. Part 69. Inter- and intramolecular hydrogen bonding effects on the structure, solubility, and reactivity of 4,5-dicarboxyimidazoles", JOURNAL OF MOLECULAR STRUCTURE., vol. 478, no. 1-3, 1 mars 1999 (1999-03-01), pages 145-154, XP055340428, NL ISSN: 0022-2860, DOI: 10.1016/S0022-2860(98)00669-3
- Arumugam Manthiram ET AL: "Rechargeable Lithium-Sulfur Batteries", Chemical Reviews, vol. 114, no. 23, 15 July 2014 (2014-07-15), pages 11751-11787, XP055580092, US ISSN: 0009-2665, DOI: 10.1021/cr500062v
- Stephen E. Burkhardt ET AL: "Li-Carboxylate Anode Structure-Property Relationships from Molecular Modeling", Chemistry of materials, vol. 25, no. 2, 3 January 2013 (2013-01-03), pages 132-141, XP055580135, ISSN: 0897-4756, DOI: 10.1021/cm302839z

## Description

La présente invention concerne le domaine des batteries secondaires au lithium, sodium, potassium ou magnésium. Elle a plus particulièrement pour objet l'utilisation de l'acide 4,5-imidazoledicarboxylique pour l'obtention d'un matériau actif d'électrodes pour ces batteries secondaires, en particulier pour des batteries lithium-ion.

Les batteries au lithium sont de plus en plus utilisées comme sources d'énergie autonome, en particulier dans les équipements portables, où ils remplacent progressivement les accumulateurs nickel-cadmium (NiCd) et nickel-hydrure métallique (NiMH). Cette évolution s'explique par l'amélioration continue des performances des accumulateurs au lithium, leur conférant ainsi des densités d'énergie nettement supérieures à celles proposées par les filières NiCd et NiMH. Les batteries au lithium trouvent de multiples applications, notamment dans les nouvelles technologies de l'information et de la communication (NTIC), des dispositifs médicaux, les véhicules électriques, le stockage de l'énergie de cellules photovoltaïques, etc.

Ces générateurs électrochimiques au lithium fonctionnent classiquement sur le principe d'insertion ou de désinsertion (ou intercalation-désintercalation) du lithium sur au moins une électrode. En particulier, dans un accumulateur lithium-ion, les cations Li⁺ font ainsi des allers-retours entre les électrodes, respectivement positive et négative, à chaque charge et décharge de l'accumulateur. Le matériau actif de l'électrode positive est capable de libérer des ions lithium au moment de la charge et d'incorporer des ions lithium au moment de la décharge.

Outre le phénomène d'insertion ou de désinsertion on peut également rencontrer des mécanismes liés à la formation d'alliages ou de réactions dites « de conversions » (réduction d'un cation métallique au degré d'oxydation 0 sous forme de nanoparticules en parallèle de la formation de LiO₂/Li₂O puis oxydo-réduction de l'oxyde de lithium catalysé par les nanoparticules métalliques).

D'une manière générale, les composés actifs d'électrodes utilisés dans les accumulateurs commerciaux sont, pour l'électrode positive, des composés lamellaires tels que LiCoO₂, LiNiO₂ et mixtes Li(Ni, Co, Mn, Al)O₂ ou des composés de structure spinelle de compositions proches de LiMn₂O₄. L'électrode négative est généralement du carbone (graphite, coke, etc.) ou éventuellement le spinelle Li₄Ti₅O₁₂ ou un métal formant un alliage avec le lithium (Sn, Si, etc.).

Pour répondre plus particulièrement aux nouveaux marchés de l'automobile hybride et électrique ou du solaire photovoltaïque, les contraintes de coût, de volume de production et de performances en puissance imposent la recherche de nouveaux matériaux actifs d'électrode.

Dans cette optique, des molécules organiques ont déjà été proposées pour leur aptitude à capter du lithium de façon réversible en libérant ou en captant un électron, telles que par exemple la phénanthraquinone, l'acide 2,5-dihydroxytéréphtalique, le téréphtalate de lithium ou encore l'hydroquinone [1].

CN 103 236 542 A décrit la préparation d'un matériau composite soufre/MOF pour une électrode positive d'une batterie lithium-soufre, le MOF ("Metal-Organic Framework") étant formé à partir de sels métalliques et de ligands organiques, par exemple à partir d'un sel de nitrate d'indium et de l'acide 4,5-imidazole-2-carboxylique. Le soufre représente le matériau actif de l'électrode positive ; le réseau MOF a uniquement un rôle structurel et n'a aucune fonction en tant que matériau actif d'électrode.

La présente invention vise à proposer de nouveaux composés utilisables comme matériau actif d'électrode pour des batteries secondaires au lithium, sodium, potassium ou magnésium, de préférence des batteries secondaires au lithium.

Ainsi, les inventeurs ont constaté que l'acide 4,5-imidazoledicarboxylique possède une activité électrochimique particulièrement intéressante pour une application comme matériau actif d'électrode, et notamment pour une batterie secondaire au lithium.

Ainsi, l'invention concerne, selon un premier de ses aspects, l'utilisation comme matériau actif d'électrode d'un composé de formule (I) : 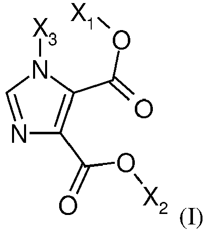
dans laquelle X₁, X₂ et X₃ identiques ou différents, représentent un atome choisi parmi l'hydrogène et le lithium, le sodium ou le potassium,
à l'état de base ou de sel ;
ainsi que ses formes tautomères.

Comme illustré dans les exemples qui suivent, de tels composés s'avèrent particulièrement avantageux comme matériau actif d'électrodes pour des batteries secondaires au lithium, au sodium, au potassium ou au magnésium, de préférence au lithium.

Avantageusement, contrairement aux électrodes classiques à base de matériaux inorganiques d'insertion pulvérulents, les électrodes comprenant les composés de l'invention peuvent être mises en forme selon des techniques classiques d'enduction ou d'impression, ou toute autre technique de mise en œuvre de matériaux organiques en solution (techniques de coating comme la tournette, le deep-coating, le spray, le drop casting) ou à l'état fondu (technique d'extrusion, d'injection, de rotomoulage, d'extrusion soufflage...).

L'invention concerne ainsi, selon un autre de ses aspects, une électrode comprenant un composé de formule (I) précitée. Elle concerne encore, selon un autre de ses aspects, un générateur électrochimique, en particulier une batterie secondaire au lithium, comprenant une telle électrode.

D'autres caractéristiques, variantes et avantages des composés mis en œuvre selon l'invention ressortiront mieux à la lecture de la description, des exemples et figures qui vont suivre, donnés à titre illustratif et non limitatif de l'invention.

Dans la suite du texte, les expressions « compris entre ... et ... » et « allant de ... à ... » et « variant de ... à ... » sont équivalentes et entendent signifier que les bornes sont incluses, sauf mention contraire.

Sauf indication contraire, l'expression « comportant/comprenant un(e) » doit être comprise comme « comportant/comprenant au moins un(e) ».

### COMPOSES DE L'INVENTION

Comme précisé ci-dessus, les composés mis en œuvre pour former un matériau actif d'électrode sont de formule (I) suivante : 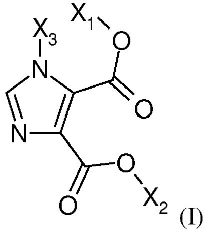 dans laquelle X_{1,} X₂ et X₃ identiques ou différents, représentent un atome choisi parmi l'hydrogène et le lithium, le sodium ou le potassium.

Il est entendu que la nature des groupements X₁, X₂ et X₃, différents d'atome(s) d'hydrogène de la formule (I) dépend de l'application envisagée, plus précisément de la nature de la batterie secondaire pour laquelle le composé est destiné à être mis en œuvre (batterie au lithium, au sodium, au potassium).

De préférence, X₁, X₂ et X₃, identiques ou différents, sont choisis parmi l'hydrogène et le lithium. De tels composés trouvent une application particulièrement intéressante comme matériau actif d'électrode dans des batteries secondaires au lithium.

La présente invention concerne également les formes tautomériques des composés de formule (I) selon l'invention.

On entend par une « une forme tautomère », un isomère de constitution dont la structure diffère par la position d'un atome, par exemple un atome d'hydrogène, et d'une ou de plusieurs liaisons multiples. Deux formes tautomères sont capables de se transformer facilement et réversiblement l'une en l'autre.

Les composés de formule (I) peuvent exister à l'état de bases ou de sels. De tels sels font également partie de l'invention.

Par exemple, les sels peuvent être des sels de cation alcalin, alcalino-terreux ou de métaux de transition, tel que par exemple le carboxylate de lithium/Na.

Selon un mode de réalisation particulièrement préféré, les radicaux X₁, X₂ et X3 du composé de formule (I) représentent des atomes d'hydrogène.

Autrement dit, le composé de formule (I) mis en œuvre comme matériau actif d'électrode est l'acide 4,5-imidazoledicarboxylique de formule (A) représentée ci-dessous :

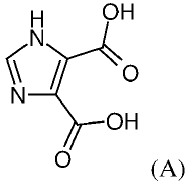

Selon un autre mode de réalisation particulier, le composé mis en œuvre selon l'invention est une forme lithiée, sodée ou potassée de la molécule acide 4,5-imidazoledicarboxylique, notamment pour une application comme matériau actif d'électrode, respectivement dans une batterie secondaire au lithium, sodium ou potassium.

Autrement dit, il s'agit d'un composé de formule (I') : 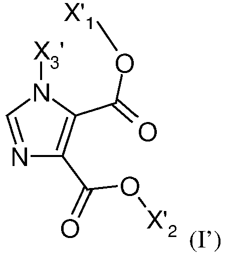
dans laquelle l'un des X'₁, X'₂ et X₃' représente un atome de lithium, de sodium ou de potassium, les deux autres, identiques ou différents, représentant un atome d'hydrogène ou un atome de lithium, de sodium ou de potassium ;
à l'état de base ou de sel, ou l'une de ses formes tautomères.

De préférence, l'un des X'₁, X'₂ et X₃' représente un atome de lithium, les deux autres, identiques ou différents, représentant un atome d'hydrogène ou de lithium. En particulier, X₁', X₂' et X₃' représentent des atomes de lithium.

Ainsi, selon un autre de ses aspects, l'invention concerne un composé de formule (I') : 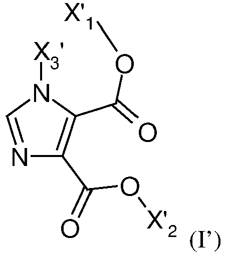
dans laquelle l'un des X'₁, X'₂ et X₃' représente un atome de lithium, de sodium ou de potassium, les deux autres, identiques ou différents, représentant un atome d'hydrogène ou un atome de lithium, de sodium ou de potassium ;
à l'état de base ou de sel, ou l'une de ses formes tautomères.

De préférence, l'un des X'₁, X'₂ et X₃' représente un atome de lithium, les deux autres, identiques ou différents, représentant un atome d'hydrogène ou de lithium. En particulier, X₁', X₂' et X₃' représentent des atomes de lithium.

Les composés de formule (I) tels que définis ci-dessus peuvent être disponibles commercialement ou préparés par des méthodes générales connues de l'homme du métier.

A titre d'exemple, l'acide 4,5-imidazoledicarboxylique (A) est disponible commercialement par exemple auprès du fournisseur Sigma Aldrich.

La forme lithiée de l'acide 4,5-imidazoledicarboxylique peut, quant à elle, être obtenue préalablement, par exemple par réaction acide-base avec une base telle que LiOH, Li₂CO₃, avec des hydrures tels que LiH ou avec un organolithien, à partir de l'acide 4,5-imidazoledicarboxylique. Alternativement, elle peut être obtenue *in situ* à partir de la mise en œuvre de l'acide 4,5-imidazoledicarboxylique pour préparer une électrode d'un générateur électrochimique, lors la première réduction.

Il en est de même pour les formes potassées et sodées qui peuvent être obtenues soit préalablement, par exemple par réaction acide-base à partir de l'acide 4,5-imidazoledicarboxylique ou *in situ* à partir de la mise en œuvre de l'acide 4,5-imidazoledicarboxylique pour préparer une électrode d'un générateur électrochimique, lors la première réduction.

Les composés selon l'invention se présentent généralement sous forme d'un solide qui se prête à une utilisation telle quelle ou par exemple en solution.

### MATERIAU ACTIF D'ELECTRODE

Comme mentionné précédemment, les composés de formule (I) sont utilisés comme matériaux actifs d'électrode.

Par « matériau actif d'électrode », on entend au sens de l'invention un matériau d'insertion/désinsertion d'un cation Cⁿ⁺ dans lequel n vaut 1 ou 2 (Li⁺, Na⁺, K⁺ ou Mg²⁺) d'une électrode d'un générateur électrochimique. Plus particulièrement, le matériau actif de l'électrode positive est capable de libérer des ions Cⁿ⁺ au moment de la charge et d'incorporer des ions Cⁿ⁺ au moment de la décharge du générateur électrochimique. Inversement, le matériau actif de l'électrode négative est capable d'incorporer des ions Cⁿ⁺ au moment de la charge et de libérer des ions Cⁿ⁺ au moment de la décharge du générateur électrochimique.

Le composé de formule (I) peut aussi bien être utilisé comme matériau actif d'électrode positive ou comme matériau actif d'électrode négative.

De préférence, il est utilisé comme matériau actif d'électrode négative, en particulier pour un générateur électrochimique, notamment pour une batterie secondaire au lithium, au sodium, au potassium ou au magnésium, de préférence au litihium.

Selon un mode de réalisation particulier, le composé de formule (I) peut être mis en œuvre conjointement avec un ou plusieurs additif(s) conducteur(s) électronique(s).

Le ou lesdits additifs conducteurs électroniques peuvent être choisis parmi des fibres de carbone, du noir de carbone, des nanotubes de carbone, du graphène et leurs analogues.

Un additif conducteur particulièrement préféré est le noir de carbone de type Super P™.

Selon un mode de réalisation préféré, le ou lesdits additif(s) conducteur(s) électronique(s) et le ou lesdits composé(s) de formule (I) peuvent être mis en œuvre dans un ratio pondéral composé(s) de formule (I)/additif(s) conducteur(s) électronique(s) compris entre 0,5 et 200, de préférence entre 1 et 20, en particulier entre 1 et 5.

Selon un autre mode de réalisation particulier, le composé de formule (I) peut être mis en œuvre conjointement avec un ou plusieurs liant(s).

De tels liants peuvent être choisi(s) parmi des liants fluorés, en particulier parmi le polytétrafluoroéthylène, le polyfluorure de vinylidène (PVDF), les polymères dérivés de carboxyméthylcellulose, les polysaccharides et les latex notamment de type caoutchouc styrène-butadiène (SBR ou en langue anglaise « styrene-butadiene rubber »).

Un liant particulièrement préféré est le poly(fluorure de vinylidène) (PVDF).

Selon un mode de réalisation préféré, le ou lesdits liants et le ou lesdits composé(s) de formule (I) peuvent être mis en œuvre dans un ratio massique composé(s) de formule (I)/liant(s) compris entre 1 et 50, de préférence entre 2 et 20.

L'invention concerne aussi, selon un autre de ses aspects, un matériau actif d'électrode comprenant au moins un composé de formule (I'), forme lithiée, sodée ou potassée de l'acide 4,5-imidazoledicarboxylique.

Dans le cadre de ce mode de réalisation particulier, un tel matériau peut éventuellement comprendre en outre un ou plusieurs aditifs conducteurs électroniques, en particulier tels que décrits précédemment.

Selon un autre de ses aspects, l'invention concerne encore un matériau actif d'électrode comprenant au moins un composé de formule (I) dans laquelle X₁, X₂ et X₃ représentent un atome d'hydrogène (autrement dit, l'acide 4,5-imidazoledicarboxylique) et au moins un additif conducteur électronique, en particulier tels que décrits précédemment.

Le matériau d'électrode peut être préparé sous la forme d'une poudre dispersée ou en solution.

Comme indiqué précédemment, le matériau d'électrode peut en outre être combiné avec un ou plusieurs composés additionnels classiquement mis en œuvre, comme par exemple un liant.

Avantageusement, ce matériau est immobilisé au niveau de l'électrode.

### Electrode

Selon encore un autre de ses aspects, l'invention concerne une électrode comprenant au moins un composé de formule (I) tel que défini précédemment et éventuellement un ou plusieurs additif(s) conducteur(s) électronique(s) et/ou liant(s), en particulier tels que décrits précédemment.

De préférence, le ou lesdits composé(s) de formule (I) représente(nt) de 10 % à 95 % en poids du poids total de l'électrode, en particulier plus de 40 % en poids, et plus particulièrement de 40 % à 80 % en poids, par rapport au poids total de ladite électrode.

Une électrode selon l'invention peut être utilisée comme électrode positive ou comme électrode négative, de préférence comme électrode négative, en particulier d'un générateur électrochimique, notamment d'une batterie secondaire au lithium.

De manière classique, une électrode selon l'invention peut comporter un collecteur de courant sur lequel est appliqué au moins un composé de formule (I), notamment *via* les techniques développées ci-dessous.

Par exemple, du cuivre, du feutre de carbone ou de l'acier inoxydable peuvent être utilisés comme collecteur de courant pour une électrode positive ; et du cuivre, ou de l'acier, traités en une feuille découpée, un métal en mousse ou une plaque de feuille laminé, par exemple, peuvent être utilisés comme collecteur de courant pour une électrode négative.

Selon un mode de réalisation particulier, une électrode selon l'invention comprend un collecteur de courant à base de cuivre, par exemple sous la forme d'une feuille de cuivre.

Comme évoqué précédemment, une électrode selon l'invention peut en outre comprendre un ou plusieurs additif(s) conducteur(s) électronique(s), notamment choisi(s) parmi les additifs listés précédemment, en particulier le noir de carbone.

Selon un mode de réalisation préféré, le ou lesdits additifs conducteurs électroniques peuvent être présents en une quantité inférieure ou égale à 60 % en poids par rapport au poids total de ladite électrode, de préférence inférieure ou égale à 40 % en poids, et plus particulièrement inférieure ou égale à 20 % en poids, par rapport au poids total de ladite électrode.

Selon un mode de réalisation particulier, une électrode selon l'invention peut comprendre un ou plusieurs liant(s), notamment choisi parmi les liants listés précédemment, en particulier le poly(fluorure de vinylidène).

Selon un mode de réalisation préféré, le ou lesdits liants peuvent être présents en une quantité inférieure ou égale à 40 % en poids, par rapport au poids total de l'électrode, de préférence inférieure ou égale à 20 % en poids, notamment inférieure ou égale à 10 % en poids, et plus particulièrement inférieure ou égale à 5 % en poids, par rapport au poids total de l'électrode.

Une électrode peut en outre comprendre d'autres additifs couramment mis en œuvre pour des électrodes de générateurs électrochimiques, en particulier pour des batteries secondaires au lithium.

### Procédé de préparation d'électrode

Une électrode selon l'invention peut être préparée *via* au moins les étapes consistant en :
(i) disposer d'un mélange formé d'au moins un composé de formule (I) tel que défini précédemment, d'une phase liquide en particulier comprenant un ou plusieurs solvants organiques ou aqueux, et éventuellement d'un ou plusieurs additif(s) conducteur(s) électronique(s) et/ou liant(s) ; et
(ii) déposer ledit mélange de l'étape (i) par enduction ou par une technique d'impression sur un substrat de base ou par extrusion ou par colaminage.

La dispersion de l'étape (i), lorsqu'elle est aqueuse, peut également comporter un épaississant, par exemple de type carboxy méthylcellulose, hydroxypropyle méthylcellulose, et/ou un surfactant et/ou un sel (LiOH par exemple). A titre d'exemple de solvant organique, la dispersion en étape (i) peut comprendre la N-méthyl-2-pyrrolidone.

Une telle dispersion est également couramment appelée « encre ». L'encre peut par exemple être déposée selon l'étape (ii) sur un collecteur de courant, tel qu'une feuille métallique, par exemple en cuivre. Le dépôt de l'encre peut par exemple être réalisé par flexographie, héligravure, sérigraphie, jet d'encre ou spray. L'homme du métier est à même d'ajuster les conditions de mise en œuvre de ces différentes techniques.

### Batterie secondaire au lithium, au sodium, au potassium ou au magnésium

Comme évoqué précédemment, la présente invention se rapporte à une batterie secondaire au lithium, au sodium, au potassium ou au magnésium, comprenant une électrode selon l'invention.

De préférence, il s'agit d'une batterie secondaire au lithium.

Il peut s'agir d'une batterie lithium métal, lithium-ion, lithium-polymère, lithium-soufre, lithium-air ou supercondensateur, et de préférence une batterie lithium-ion.

Le reste de la batterie peut être formée selon des méthodes conventionnelles.

De manière générale, les batteries lithium-ion présentent une architecture avec deux électrodes (une électrode positive et une électrode négative), toutes les deux revêtues sur un collecteur de courant conducteur électrique, disposées de part et d'autre d'un séparateur organique ou inorganique. Les deux techniques de montage de cette architecture actuellement les plus utilisées sont le bobinage (enroulement des différents constituants dans une géométrie cylindrique ou prismatique) et le stack (empilement couche par couche des différents éléments). Bien entendu, d'autres techniques de montage pour former une batterie sont envisageables, telles que les techniques de l'impression.

L'électrode selon l'invention peut constituer l'électrode positive ou l'électrode négative de la batterie. De préférence, elle constituera l'électrode négative.

L'invention va maintenant être décrite au moyen des figures et exemples suivants donnés bien entendu à titre illustratif et non limitatif de l'invention.

### FIGURES

Figure 1 : Courbes de voltampérométrie cyclique d'une pile bouton selon l'invention obtenues selon l'exemple 2 (anode : lithium métallique ; cathode : acide 4,5-imidazoledicarboxylique:SuperP:Liant = 40:40:20 (massique) sur un collecteur de courant en cuivre) ;
Figure 2 : Courbes de voltampérométrie cyclique d'une pile bouton non conforme à l'invention, obtenues selon l'exemple 2 (anode : lithium métallique ; cathode : SuperP:Liant = 2 :1 (massique) sur un collecteur de courant en cuivre) ;
Figure 3 : Capacités spécifiques en fonction du nombre de cycles (tests effectués à C/20 entre 0,1 et 3 V) obtenues selon l'exemple 3 pour une pile bouton dans le cas où la cathode comprend l'acide 4,5-imidazoledicarboxylique (figure 3a) et dans le cas où la cathode comprend uniquement du noir de carbone (figure 3b).

### EXEMPLES

### EXEMPLE 1

### Préparation d'une électrode selon l'invention

Une électrode ayant la composition acide 4,5-imidazoledicarboxylique:SuperP:Liant = 40:40:20 (en % massique) est préparée selon les étapes suivantes.

40 % (en masse) d'acide 4,5-imidazoledicarboxylique, 40% de carbone de type Super P™ et 20 % de poly(fluorure de vinylidène) (PVDF) sont mélangés avec de la N-méthyl-2-pyrrolidone afin de fabriquer une encre.

Cette encre est ensuite enduite à 100 µm d'épaisseur (humide) sur une feuille de cuivre, puis est séchée dans une étuve à 60°C pendant une nuit.

Le lendemain, des disques de 14 mm de diamètre sont découpés puis placés sous presse à 10 tonnes (étape dite de calandrage). Les disques sont ensuite mis à sécher sous vide pendant 48 h à une température de 80°C.

Les électrodes ainsi obtenues sont testées dans un accumulateur lithium-métal de type « pile bouton » comme décrit en exemple 2.

### EXEMPLE 2

### Fabrication d'une pile bouton selon l'invention

Afin de déterminer les performances électrochimiques du matériau d'électrode selon l'invention, un accumulateur lithium-métal de type « pile bouton » est réalisé avec :
- une électrode négative (anode) de lithium métallique (disque de lithium de 16 mm de diamètre découpé dans un feuillard de 180 µm) ;
- une électrode positive (cathode) telle que préparée en exemple 1, comprenant l'acide 4,5-imidazoledicarboxylique ; et
- un séparateur de type Celgard 2400® (séparateur de membrane microporeux) imbibé d'un électrolyte de type LP 30® (1M de LiPF₆ en solution dans un mélange de carbonate d'éthylène et de carbonate de diméthyle).

### EXEMPLE 3

### Efficacité d'une pile-bouton intégrant une électrode selon l'invention

### Voltampérométrie cyclique

La pile bouton préparée en exemple 2 est analysée en voltampérométrie cyclique afin d'observer les phénomènes électrochimiques ayant lieu dans la batterie.

Les courbes de voltampérométrie cyclique sont mesurées en balayant le potentiel de l'électrode de travail (comprenant le matériau de l'invention) entre 0,1 et 3 V *vs* Li+/Li° (le lithium métal servant à la fois de contre-électrode et d'électrode de référence) à une vitesse de 0,1 mV/s.

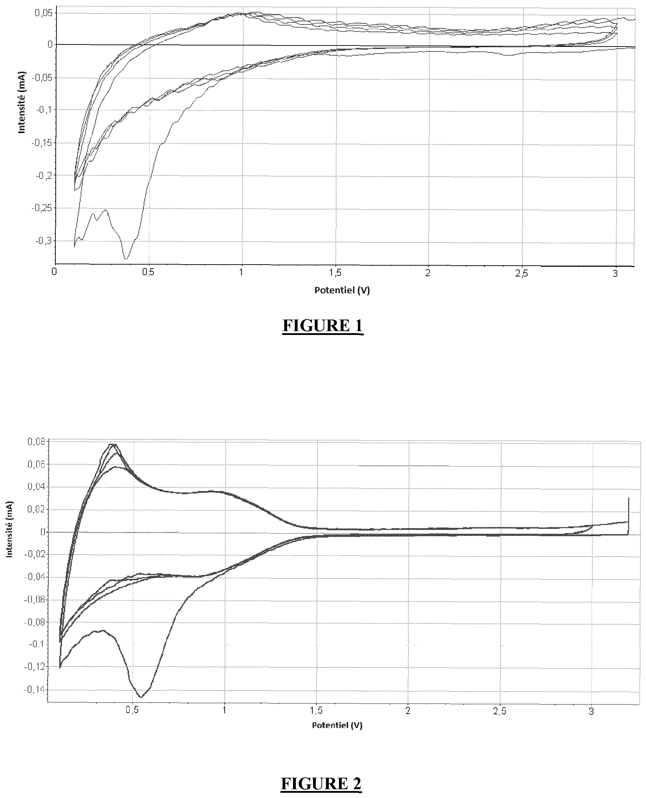

Les résultats obtenus (Figure 1) en voltampérométrie cyclique montrent que la pile bouton à base d'acide 4-5 imidazoledicarboxilique a une activité électrochimique dans la plage de 0,1 V à 3 V *vs* Li+/Li°. En effet, un courant important, en réduction d'une part et en oxydation d'autre part, est détecté sur cette gamme de potentiel démontrant l'activité électrochimique partiellement réversible du composé de l'invention.

Il convient de noter que, dans cette gamme de tension 0,1 V à 3 V, le carbone utilisé comme additif conducteur, peut également insérer du lithium.

A titre de comparaison, une électrode est préparée comme décrit en exemple 1 sans introduire l'acide 4,5-imidazoledicarboxylique. Cette électrode a donc la composition SuperP:liant = 80:20 (en % massique). Une pile bouton intégrant comme cathode une telle électrode est préparée comme décrit précédemment.

Les courbes de voltampérométrie cyclique sont représentées en figure 2, et mettent en évidence que le carbone de type SuperP™ présente une activité électrochimique dans la plage de 0,1 V à 3 V *vs* Li+/Li°.

### Tests galvanostatistiques

De manière à pouvoir séparer la capacité, induite par la molécule organique d'acide 4-5 imidazoledicarboxilique, du carbone servant d'additif dans la fabrication de l'électrode, des tests galvanostatiques ont été effectués dans les bornes de potentiel 0,1 à 3 V à un régime de C/20 (régime d'une charge et d'une décharge en 20 heures).

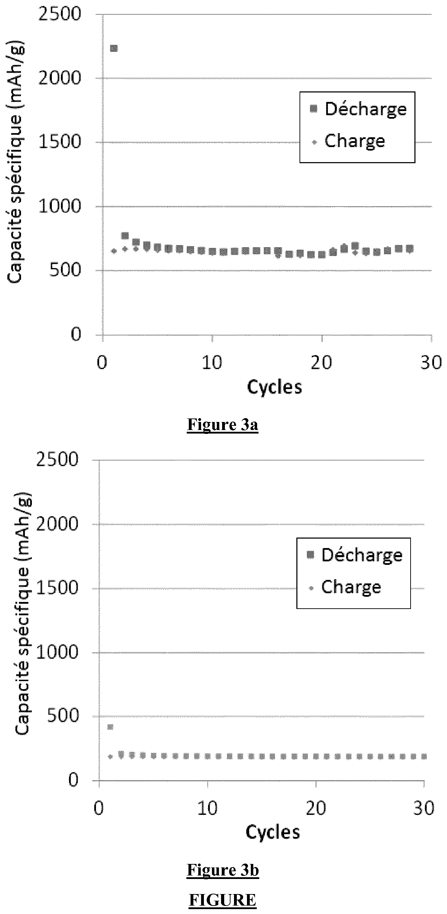

Les graphes en figure 3 représentent les capacités spécifiques obtenues avec la pile bouton selon l'invention préparée en exemple 2 (figure 3a) et avec une pile-bouton, intégrant une électrode comprenant uniquement le carbone à titre d'additif conducteur et dénué d'acide 4,5-imidazoledicarboxylique, préparée à titre de comparaison, comme décrit en exemple 2 (figure 3b).

Pour ces deux piles, la capacité spécifique a donc été mesurée en fonction du nombre de cycles, en régime de charge et de décharge.

Dans les deux cas, les résultats mettent en évidence une première décharge très importante qui correspond à une réduction irréversible du matériau actif et/ou à diverses réactions électrochimiques irréversibles telles que la décomposition de l'électrolyte à bas potentiel sur la surface de l'électrode (majoritairement développée par le Super P).

Par ailleurs, les résultats montrent très clairement que la capacité spécifique obtenue est nettement supérieure lorsque le matériau actif comprend de l'acide 4,5-imidazoledicarboxylique (capacité stabilisée à 660 mAh/g) comparativement au cas où l'électrode comprend uniquement du noir de carbone (capacité stabilisée à 187 mAh/g).

L'acide 4,5-imidazoledicarboxylique possède donc une activité électrochimique élevée intéressante pour des applications de types batteries Li-ion organique.

### Référence

[1] Liang et al. « Organic Electrode Materials for Rechargeable Lithium Batteries », Adv. Energy Mater., 2012, 2, 742-769.

## Revendications

1. Utilisation comme matériau actif d'électrode d'un composé de formule (I) : 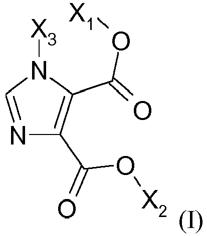
dans laquelle X₁, X₂ et X₃, identiques ou différents, représentent un atome choisi parmi l'hydrogène et le lithium, le sodium ou le potassium,
à l'état de base ou de sel ;
ainsi que ses formes tautomères.

2. Utilisation selon la revendication 1, **caractérisée en ce que** X₁, X₂ et X₃, identiques ou différents, représentent un atome choisi parmi l'hydrogène et le lithium.

3. Utilisation selon la revendication 1 ou 2, **caractérisée en ce que** le composé de formule (I) est l'acide 4,5-imidazoledicarboxylique.

4. Utilisation selon l'une quelconque des revendications précédentes, **caractérisée en ce que** ledit composé de formule (I) est mis en œuvre conjointement avec au moins un additif conducteur électronique, en particulier choisi parmi des fibres de carbone, du noir de carbone, des nanotubes de carbone, du graphène et leurs analogues.

5. Utilisation selon l'une quelconque des revendications précédentes, **caractérisée en ce que** ledit composé de formule (I) est mis en œuvre conjointement avec au moins un liant, en particulier choisi parmi des liants fluorés, en particulier parmi le polytétrafluoroéthylène, le polyfluorure de vinylidène, les polymères dérivés de carboxyméthylcellulose, les polysaccharides et les latex notamment de type caoutchouc styrène-butadiène.

6. Utilisation selon l'une quelconque des revendications précédentes, comme matériau actif d'électrode négative, en particulier pour une batterie secondaire au lithium, au sodium, au potassium ou au magnésium, de préférence au lithium.

7. Composé de formule (I') : 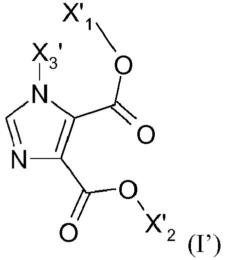
dans laquelle l'un des X'₁, X'₂ et X₃' représente un atome de lithium ou de potassium, les deux autres, identiques ou différents, représentant un atome d'hydrogène ou un atome de lithium, de sodium ou de potassium;
à l'état de base ou de sel, ou l'une de ses formes tautomères.

8. Composé de formule (I') selon la revendication 7, dans lequel l'un des X'₁, X'₂ et X₃' représente un atome de lithium, les deux autres, identiques ou différents, représentant un atome d'hydrogène ou de lithium, en particulier X₁', X₂' et X₃' représentent des atomes de lithium.

9. Matériau actif d'électrode comprenant au moins un composé de formule (I') tel que défini en revendication 7 ou 8 et, éventuellement, un ou plusieurs additif(s) conducteur(s) électronique(s), en particulier tel(s) que défini(s) en revendication 4.

10. Matériau actif d'électrode comprenant au moins un composé de formule (I) tel que défini en revendication 3 et au moins un additif conducteur électronique, en particulier tel que défini en revendication 4.

11. Matériau selon la revendication 9 ou 10, se présentant sous la forme d'une poudre dispersée ou en solution.

12. Electrode comprenant un composé de formule (I) tel que défini en l'une quelconque des revendications 1 à 3, ladite électrode comportant en particulier un collecteur de courant sur lequel est appliqué au moins ledit composé de formule (I).

13. Electrode selon la revendication 12, comprenant un ou plusieurs additif(s) conducteur(s) électronique(s), en particulier tel(s) que défini(s) en revendication 4 et/ou un ou plusieurs liant(s) en particulier tel(s) que défini(s) en revendication 5.

14. Procédé de préparation d'une électrode telle que définie selon la revendication 12 ou 13, comprenant au moins les étapes consistant en :
(i) disposer d'un mélange formé d'au moins un composé de formule (I) tel que défini en l'une quelconque des revendications 1 à 3, d'une phase liquide, en particulier comprenant un ou plusieurs solvants organiques ou aqueux, et éventuellement d'un ou plusieurs additif(s) conducteur(s) électronique(s) et/ou liant(s) ; et
(ii) déposer ledit mélange de l'étape (i) par enduction ou par une technique d'impression sur un substrat de base ou par extrusion ou par colaminage.

15. Batterie secondaire au lithium, au sodium, au potassium ou au magnésium comprenant une électrode telle que définie selon la revendication 12 ou 13, ladite batterie étant en particulier une batterie au lithium, de préférence choisie parmi une batterie lithium-métal, lithium-ion, lithium-polymère, lithium-soufre, lithium-air et supercondensateur, en particulier une batterie lithium-ion.

## Patentansprüche

1. Verwendung einer Verbindung der Formel (I) 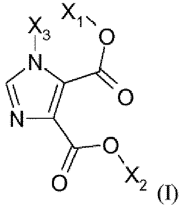
in der X₁, X₂ und X₃ gleich oder verschieden sind und für ein aus Wasserstoff und Lithium, Natrium oder Kalium ausgewähltes Atom stehen,
in Basen- oder Salzform
sowie den tautomeren Formen davon als Elektrodenaktivmaterial.

2. Verwendung nach Anspruch 1, **dadurch gekennzeichnet, dass** X₁, X₂ und X₃ gleich oder verschieden sind und für ein aus Wasserstoff und Lithium ausgewähltes Atom stehen.

3. Verwendung nach Anspruch 1 oder 2, **dadurch gekennzeichnet, dass** es sich bei der Verbindung der Formel (I) um 4,5-Imidazoldicarbonsäure handelt.

4. Verwendung nach einem der vorhergehenden Ansprüche, **dadurch gekennzeichnet, dass** die Verbindung der Formel (I) zusammen mit mindestens einem elektronenleitenden Additiv, das insbesondere aus Kohlefasern, Ruß, Kohlenstoffnanoröhren, Graphen und Analogen davon ausgewählt ist, verwendet wird.

5. Verwendung nach einem der vorhergehenden Ansprüche, **dadurch gekennzeichnet, dass** die Verbindung der Formel (I) zusammen mit mindestens einem Bindemittel, das insbesondere aus fluorierten Bindemitteln, die insbesondere aus Polytetrafluorethylen und Polyvinylidenfluorid ausgewählt sind, Polymeren, die sich von Carboxymethylcellulose ableiten, Polysacchariden und Latices, insbesondere von Styrol-Butadien-Kautschuk-Typ, ausgewählt ist, verwendet wird.

6. Verwendung nach einem der vorhergehenden Ansprüche, als Negativelektroden-Aktivmaterial, insbesondere für eine Lithium-, Natrium-, Kalium- oder Magnesium-Sekundärbatterie, vorzugsweise eine Lithium-Sekundärbatterie.

7. Verbindung der Formel (I'): 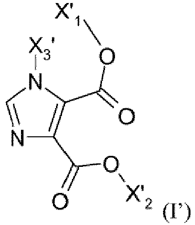
in der eines von X'₁, X'₂ und X'₃ für ein Lithium- oder Kaliumatom steht und die beiden anderen gleich oder verschieden sind und für ein Wasserstoffatom oder ein Lithium-, Natrium- oder Kaliumatom stehen;
in Basen- oder Salzform oder eine der tautomeren Formen davon.

8. Verbindung der Formel (I') nach Anspruch 7, wobei eines von X'₁, X'₂ und X'₃ für ein Lithiumatom steht und die beiden anderen gleich oder verschieden sind und für ein Wasserstoff- oder Lithiumatom stehen, insbesondere X'₁, X'₂ und X'₃ für Lithiumatome stehen.

9. Elektrodenaktivmaterial, umfassend mindestens eine Verbindung der Formel (I') gemäß Anspruch 7 oder 8 und gegebenenfalls ein oder mehrere elektronenleitende Additive, insbesondere gemäß Anspruch 4.

10. Elektrodenaktivmaterial, umfassend mindestens eine Verbindung der Formel (I) gemäß Anspruch 3 und mindestens ein elektronenleitendes Additiv, insbesondere gemäß Anspruch 4.

11. Material nach Anspruch 9 oder 10, das in Form eines dispergierten Pulvers oder in Lösung vorliegt.

12. Elektrode, umfassend eine Verbindung der Formel (I) gemäß einem der Ansprüche 1 bis 3, wobei die Elektrode insbesondere einen Stromsammler umfasst, auf denen mindestens die Verbindung der Formel (I) aufgebracht ist.

13. Elektrode nach Anspruch 12, umfassend ein oder mehrere elektronenleitende Additive, insbesondere gemäß Anspruch 4, und/oder ein oder mehrere Bindemittel, insbesondere gemäß Anspruch 5.

14. Verfahren zur Herstellung einer Elektrode gemäß Anspruch 12 oder 13, umfassend mindestens die Schritte, die aus Folgendem bestehen:
(i) Bereitstellen einer Mischung von mindestens einer Verbindung der Formel (I) gemäß einem der Ansprüche 1 bis 3, einer flüssigen Phase, die insbesondere ein oder mehrere organische oder wässrige Lösungsmittel umfasst, und gegebenenfalls einem oder mehreren elektronenleitenden Additiven und/oder Bindemitteln und
(ii) Abscheiden der Mischung aus Schritt (i) durch Beschichten oder durch eine Drucktechnik auf einem Basissubstrat oder durch Extrusion oder durch Co-Laminierung.

15. Lithium-, Natrium-, Kalium- oder Magnesium-Sekundärbatterie, umfassend eine Elektrode gemäß Anspruch 12 oder 13, wobei es sich bei der Batterie insbesondere um eine Lithiumbatterie handelt, die vorzugsweise aus einer Lithium-Metall-, Lithium-Ionen-, Lithium-Polymer-, Lithium-Schwefel-, Lithium-Luft- und Superkondensator-Batterie, insbesondere einer Lithium-Ionen-Batterie, ausgewählt ist.

## Claims

1. Use, as electrode active material, of a compound of formula (I): 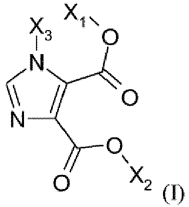
in which X₁, X₂ and X₃, which are identical or different, represent an atom chosen from hydrogen and lithium, sodium or potassium,
in the form of a base or salt,
and also its tautomeric forms.

2. Use according to Claim 1, **characterized in that** X₁, X₂ and X₃, which are identical or different, represent an atom chosen from hydrogen and lithium.

3. Use according to Claim 1 or 2, **characterized in that** the compound of formula (I) is 4,5-imidazoledicarboxylic acid.

4. Use according to any one of the preceding claims, **characterized in that** said compound of formula (I) is used jointly with at least one electron-conducting additive, in particular chosen from carbon fibres, carbon black, carbon nanotubes, graphene and their analogues.

5. Use according to any one of the preceding claims, **characterized in that** said compound of formula (I) is used jointly with at least one binder, in particular chosen from fluorinated binders, in particular from polytetrafluoroethylene, polyvinylidene fluoride, polymers derived from carboxymethylcellulose, polysaccharides and latexes in particular of the styrene-butadiene rubber type.

6. Use according to any one of the preceding claims, as negative electrode active material, in particular for a lithium, sodium, potassium or magnesium, preferably lithium, secondary battery.

7. Compound of formula (I'): 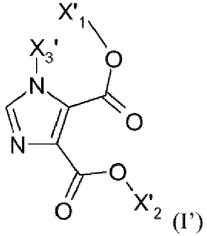
in which one of X'₁, X'₂ and X₃' represents a lithium or potassium atom, the other two, which are identical or different, representing a hydrogen atom or a lithium, sodium or potassium atom;
in the form of a base or salt, or one of its tautomeric forms.

8. Compound of formula (I') according to Claim 7, wherein one of X'₁, X'₂ and X₃' represents a lithium atom, the other two, which are identical or different, representing a hydrogen or lithium atom, in particular X'₁, X'₂ and X₃' represent lithium atoms.

9. Electrode active material comprising at least one compound of formula (I') as defined in Claim 7 or 8 and optionally one or more electron-conducting additives, in particular as defined in Claim 4.

10. Electrode active material comprising at least one compound of formula (I) as defined in claim 3 and at least one electron-conducting additive, in particular as defined in Claim 4.

11. Material according to Claim 9 or 10, provided in the form of a dispersed powder or in solution.

12. Electrode comprising a compound of formula (I) as defined in any one of Claims 1 to 3, said electrode including in particular a current collector on which at least said compound of formula (I) is applied.

13. Electrode according to Claim 12, comprising one or more electron-conducting additives, in particular as defined in Claim 4 and/or one or more binders, in particular as defined in Claim 5.

14. Process for preparing an electrode as defined according to Claim 12 or 13, comprising at least the steps consisting in:
(i) providing a mixture formed of at least one compound of formula (I) as defined in any one of Claims 1 to 3, of a liquid phase, in particular comprising one or more organic or aqueous solvents, and optionally of one or more electron-conducting additives and/or binders; and
(ii) depositing said mixture from step (i) by coating or by a technique of printing onto a base substrate or by extrusion or by corolling.

15. Lithium, sodium, potassium or magnesium secondary battery comprising an electrode as defined according to Claim 12 or 13, said battery in particular being a lithium battery, preferably chosen from a lithium-metal, lithium-ion, lithium-polymer, lithium-sulfur, lithium-air and supercapacitor battery, in particular a lithium-ion battery.
